# NEUE EUROPÄISCHE PATENTSCHRIFT

(11) **EP 3 837 094 B2**
(45) Veröffentlichungstag und Bekanntmachung des Hinweises auf die Entscheidung über den Einspruch: **24.06.2026**
(45) Hinweis auf die Patenterteilung: 27.09.2023
(21) Anmeldenummer: 19749636.7
(22) Anmeldetag: 25.07.2019
(51) Int. Cl.: B25J 9/00

(54) **VORRICHTUNG ZUM UNTERSTÜTZEN WENIGSTENS EINES ARMES EINES BENUTZERS**
DEVICE FOR SUPPORTING AT LEAST ONE ARM OF A USER
DISPOSITIF DE SOUTIEN D'AU MOINS UN BRAS D'UN UTILISATEUR

(30) Priorität: 14.08.2018 DE 102018119755
(43) Veröffentlichungstag der Anmeldung: 23.06.2021
(73) Patentinhaber: Ottobock SE & Co. KGaA, 37115 Duderstadt (DE)
(72) Erfinder: KROLL-ORYWAHL, Olaf, 37154 Northeim (DE); HARDT, Alexander, 35614 Asslar (DE)
(74) Vertreter: Gramm, Lins & Partner Patent- und Rechtsanwälte PartGmbB
(86) Internationale Anmeldenummer: PCT/EP2019/070114
(87) Internationale Veröffentlichungsnummer: WO 2020/035284

(56) Entgegenhaltungen:
- EP-A1- 3 504 033
- WO-A1-2017/157941
- WO-A2-2014/093408
- JP-A- H04 304 979
- NL-B1- 2 015 541
- US-A1- 2010 059 652
- US-A1- 2014 033 391
- US-A1- 2016 081 871
- US-A1- 2016 339 583
- US-A1- 2019 083 350

## Beschreibung

Die Erfindung betrifft eine Vorrichtung zum Unterstützen wenigstens eines Armes eines Benutzers, wobei die Vorrichtung wenigstens ein Armstützelement mit je einer Armschale zum Anlegen an jeweils einen Arm, wenigstens einen passiven Aktuator, der eingerichtet ist, eine Kraft auf wenigstens eines der Armstützelemente auszuüben, und wenigstens ein Gegenlager für die aufzubringende Kraft aufweist, das wenigstens ein Kraftübertragungselement und ein Gegenlagerelement aufweist, wobei die Vorrichtung wenigstens einen Kraftangriffshebel aufweist, der mit einem der Armstützelemente drehfest verbunden ist und einen Winkel einschließt, an dem der wenigstens eine passive Aktuator angreift.

Eine derartige Vorrichtung ist beispielsweise aus der Figur 20A,20B der US 2016/0081871 A1 bekannt.

Vorrichtungen, mit denen Arme unterstützt werden können, sind aus unterschiedlichen Dokumenten bekannt. Die in Figur 20A,20B der US 2016/0081871 A1 beschriebene Vorrichtung verfügt beispielsweise über ein Gegenlagerelement, das um den Rumpf des Benutzers herumlegbar ausgebildet ist. An ihm befinden sich Stützstreben, die seitlich neben den Schultern des Benutzers mit jeweils einem Gelenk verbunden sind, so dass der Arm angehoben werden kann. Dabei handelt es sich um die Kraftübertragungselemente. An Kraftangriffshebeln der entsprechenden Gelenke sind Federelemente angeordnet, durch die eine nach oben gerichtete Kraft auf die Armschalen ausgeübt werden kann, so dass beispielsweise beim Heben schwerer Gegenstände oder beim Arbeiten über Kopf eine Unterstützung der Arme erfolgen kann. Sollen die Arme gesenkt werden, muss durch die Arme ein Druck auf die Armschalen ausgeübt werden, der die von den Federelementen aufgebrachte Kraft übersteigt, so dass die Arme absinken.

Aus der WO 2014/0093804 A1 und der US 9,427,865 B2 ist eine ähnliche Vorrichtung bekannt, bei der als mechanischer Energiespeicher, der als passiver Aktuator wirkt, jeweils eine Feder, insbesondere eine Zugfeder vorgesehen ist, die mit einem Bowdenzug verbunden ist. Der Bowdenzug wird über eine Umlenkrolle geführt, so dass beim Verschwenken eines Armes, was eine Bewegung des Armstützelementes relativ zum Gegenlagerelement bedeutet, die Feder gedehnt wird, so dass der mechanische Energiespeicher mit Energie aufgeladen wird.

Aus der EP 3 156 193 A1 ist eine aktive Vorrichtung, die Arme beim Arbeiten über Kopf unterstützt, bekannt. Die Armschalen sind über eine Vielzahl verschiedener Gelenke und Verbindungsrahmenelemente miteinander verbunden. Dadurch sollen möglichst viele der Bewegungen, die ein Schultergelenk durchführen kann, auch mit der angelegten Vorrichtung möglich sein. Diese Vorrichtung ist jedoch aufgrund der Vielzahl an Elementen groß, konstruktionsaufwendig und damit teuer. Weitere Unterstützungsvorrichtungen, die insbesondere beim Heben von schweren Gegenständen oder beim Arbeiten über Kopf unterstützen, sind aus der WO 2014/195373 A1 und der US 2016/339583 A1 bekannt.

Nachteilig bei allen genannten Vorrichtungen ist, dass die von dem wenigstens einen passiven Aktuator auf das Armstützelement ausübbare Kraft zwar gegebenenfalls vom Winkel zwischen dem Armstützelement und dem Kraftübertragungselement abhängig ist, und die Größe der Kraft gegebenenfalls einstellbar ist, der Kraftverlauf ist jedoch durch die Konstruktion der jeweiligen Vorrichtung festgelegt.

Dies ist beispielsweise von Nachteil, wenn die Vorrichtung nacheinander von zwei unterschiedlich großen Benutzern verwendet werden soll. Während ein kleinerer Benutzer zum Arbeiten an einem bestimmten Werkstück die Arme ganz über den Kopf heben muss, muss ein größerer Benutzer die Arme lediglich teilweise anheben. Nachteilig ist die Konstruktion aus dem Stand der Technik zudem, wenn ein einzelner Benutzer unterschiedliche Tätigkeiten in unterschiedlichen Höhen ausführen soll oder die Benutzer der Vorrichtung unterschiedlich stark sind, so dass sie unterschiedlich starke Unterstützung brauchen. Der Erfindung liegt daher die Aufgabe zugrunde, diese Nachteile zu beheben oder zumindest zu mindern.

Die Erfindung löst die gestellte Aufgabe durch eine Vorrichtung zum Unterstützen wenigstens eines Armes eines Benutzers gemäß dem Oberbegriff des Anspruchs 1, die sich dadurch auszeichnet, dass der Winkel zwischen dem Kraftangriffshebel und dem Armstützelement oder dem Kraftangriffshebel und dem Kraftübertragungselement einstellbar ist, wobei der Kraftangriffshebel in einen ersten Zustand, in dem er relativ zu dem Armstützelement und dem Kraftübertragungselement schwenkbar ist, so dass der Winkel einstellbar ist, indem er Kraftangriffshebel verschwenkt wird, und in einen zweiten Zustand bringbar ist, in dem er drehfest an dem Armstützelement angeordnet ist, so dass die Position des Arms des Benutzers veränderbar ist, in der der Kraftangriffshebel senkrecht auf der durch den wenigstens einen Aktuator aufbringbaren Kraft steht, wobei sich beim Anheben des Arms des Benutzers der Winkel zwischen dem Kraftangriffshebel und dem Kraftübertragungselement verändert und sich gleichzeitig der Winkel zwischen dem Kraftangriffshebel und der Wirkrichtung der durch den wenigstens einen Aktuator aufbringbaren Kraft verändert.

Durch den wenigstens einen passiven Aktuator kann eine Kraft auf das Armstützelement ausgeübt werden, wobei diese Kraft an einem Kraftangriffshebel angreift. Der Kraftangriffshebel ist erfindungsgemäß mit einem der Armstützelemente, auf die die vom Aktuator aufbringbare Kraft ausgeübt werden soll, drehfest verbunden. Der Aktuator kann beispielsweise eine Feder oder ein elastisches Element sein, dessen eines Ende beispielsweise am Gegenlager angeordnet ist. Das andere Ende greift am Kraftangriffshebel an. Vorteilhafterweise ist das Armstützelement schwenkbar am Kraftübertragungselement angeordnet. Da der Kraftübertragungshebel drehfest am Armelement angeordnet ist, ist folglich auch der Kraftübertragungshebel schwenkbar am Kraftübertragungselement befestigt. Durch die vom passiven Aktuator auf den Kraftübertragungshebel wirkende Kraft wird folglich ein Drehmoment auf das Armstützelement und damit eine unterstützende Kraft ausgeübt. Dieses Drehmoment ist am größten, wenn der Kraftübertragungshebel senkrecht auf der Richtung der wirkenden Kraft steht.

Hebt ein Benutzer einer derartigen Vorrichtung den Arm, wird das Armstützelement und damit auch der drehfest mit ihm verbundene Kraftangriffshebel um das Kraftübertragungselement herum verschwenkt. Der Winkel zwischen dem Kraftangriffshebel und dem Kraftübertragungselement verändert sich folglich. Gleichzeitig verändert sich auch der Winkel zwischen dem Kraftangriffshebel und der Wirkrichtung der durch den wenigstens einen passiven Aktuator aufbringbaren Kraft. Bei einer bestimmten Stellung der Arme steht der Kraftangriffshebel senkrecht auf der Richtung der wirkenden Kraft. In diesem Moment ist das durch den Aktuator auf den Kraftangriffshebel und damit auf das Armstützelement aufgebrachte Drehmoment am größten und somit die Unterstützungskraft für den Arm und das Armstützelement maximal.

Erfindungsgemäß ist nun vorgesehen, dass der Winkel zwischen dem Armstützelement und dem Kraftangriffshebel einstellbar ist. Der Winkel kann folglich verändert werden und ist in wenigstens zwei, vorzugsweise vielen, insbesondere vorteilhaft stufenlos, feststellbar, so dass eine drehfeste Verbindung zwischen dem Kraftangriffshebel und dem Armstützelement erreicht wird. Durch die Einstellung dieses Winkels lässt sich die Position der Arme des Benutzers verändern, in der der Kraftangriffshebel senkrecht auf der durch den wenigstens einen Aktuator aufbringbaren Kraft steht.

In einer anderen, nicht erfindungsgemäßen Ausführungsform ist der Kraftangriffshebel drehfest am Kraftübertragungselement befestigt. Auch in diesem Fall kann der Aktuator beispielsweise eine Feder oder ein elastisches Element sein, dessen eines Ende nun jedoch nicht am Kraftübertragungselement oder dem Gegenlager angeordnet ist, sondern mit dem Armstützelement verbunden ist. Der Aktuator ist mit dem anderen Ende mit dem Kraftangriffshebel verbunden. Auch in diesem Fall kann durch den wenigstens einen passiven Aktuator ein Drehmoment auf den Kraftangriffshebel ausgeübt werden, so dass eine unterstützende Kraft auf das Armstützelement ausgeübt wird. Auch in diesem Fall ist es von Vorteil, wenn das Armstützelement drehbar am Kraftübertragungselement angeordnet ist.

Auch in dieser Ausgestaltung ist das durch den wenigstens einen passiven Aktuator erzeugte Drehmoment und damit die auf das Armstützelement ausübbare Kraft maximal, wenn der Winkel zwischen dem Kraftangriffshebel und der durch den Aktuator ausübbaren wirkenden Kraft 90° ist. Ist nun der Winkel zwischen dem Kraftangriffshebel und dem Kraftübertragungselement einstellbar, lässt sich auch in dieser Ausgestaltung diese Position, in der das Drehmoment und damit die Unterstützungskraft maximal ist, verändern.

Wird bezüglich der vorliegenden Anmeldung von einer Richtung des Kraftangriffshebels oder einem Winkel zwischen einem Bauteil und dem Kraftangriffshebel gesprochen, ist mit dieser Richtung immer die Richtung vom Kraftangriffspunkt, also dem Punkt, an dem die vom Aktuator ausübbare Kraft am Kraftangriffshebel angreift, und dem Drehpunkt gemeint, um den das Armstützelement relativ zum Kraftübertragungselement schwenkbar ist.

Erfindungsgemäß ist der Kraftangriffshebel in einem ersten Zustand, in dem er relativ zu dem Armstützelement und dem Kraftübertragungselement schwenkbar ist, und in einen zweiten Zustand bringbar, in dem er drehfest mit dem Armstützelement angeordnet ist. Während sich der Kraftangriffshebel im ersten Zustand befindet, wird ein Winkel zwischen dem Kraftangriffshebel und dem Armstützelement einstellbar. Er kann durch Verschwenken des Kraftangriffshebels verändert werden. Ist der gewünschte Winkel eingestellt, wird der Kraftangriffshebel in den zweiten Zustand überführt, so dass er nicht mehr relativ zum Armstützelement verdreht werden kann.

Vorzugsweise weist die Vorrichtung zwei Armstützelemente mit jeweils einer Armschale zum Anlegen an jeweils einen Arm des Benutzers auf.

In einer bevorzugten Ausgestaltung verfügt das Kraftübertragungselement und/oder das Armstützelement über zwei Bauteile, die durch ein Schwenkgelenk miteinander verbunden sind, so dass ein Winkel zwischen diesen beiden Bauteilen einstellbar ist. Vorteilhafterweise ist das Schwenkgelenk lösbar und feststellbar. Durch diese Ausgestaltung des Schwenkgelenkes kann eine Richtung des Kraftübertragungselementes verändert werden. Die Richtung des Kraftübertragungselementes, die beispielsweise wichtig ist, um den Winkel zwischen dem Kraftübertragungselement und dem Kraftangriffshebel zu bestimmen, ist die Richtung zwischen dem Drehpunkt, um den das Armstützelement relativ zum Kraftübertragungselement verschwenkbar ist, und dem Lagerpunkt, an dem das Kraftübertragungselement am Gegenlagerelement angeordnet ist.

In einer bevorzugten Ausgestaltung handelt es sich bei dem Gegenlagerelement des Gegenlagers beispielsweise um einen Hüftgurt, der vom Benutzer der Vorrichtung um die Hüfte oder den Bauch herum getragen wird. Das Kraftübertragungselement ist beispielsweise eine aus einem oder mehreren Bauteilen bestehende Stange, wobei zwischen mehreren Bauteilen der Stange ein oder mehrere Schwenkgelenke angeordnet sein können, durch die der Winkel zwischen den einzelnen Bauteilen einstellbar aber feststellbar ist. Dieses Kraftübertragungselement ist mit einem Ende am Gegenlagerelement befestigt. Am anderen Ende ist um ein Schwenkgelenk schwenkbar das Armstützelement angeordnet, das beispielsweise ein Abstandselement in Form einer Stange, die beispielsweise teleskopierbar oder nicht teleskopierbar ausgebildet sein kann. Alle Richtungen, die zur Bestimmung des Winkels, der erfindungsgemäß einstellbar sein soll, verwendet werden, sind in Richtung auf den jeweiligen Drehpunkt dieses Schwenkgelenkes gerichtet. Ist der Kraftangriffshebel drehfest mit dem Armstützelement verbunden, wird der Winkel gebildet zwischen der Richtung zwischen dem Kraftangriffspunkt am Kraftangriffshebel und dem Drehpunkt des Schwenkgelenkes einerseits und der Richtung des Kraftübertragungselementes andererseits, die durch die Verbindungslinie zwischen dem Drehpunkt des Schwenkgelenkes einerseits und dem Lagerpunkt andererseits gebildet wird, an dem das Kraftübertragungselement am Gegenlager positioniert ist.

Ist hingegen der Kraftangriffshebel drehfest mit dem Kraftübertragungselement verbunden, wird der Winkel bestimmt zwischen der Richtung des Kraftangriffshebels, also der Richtung zwischen dem Kraftangriffspunkt und dem Drehpunkt des Schwenkgelenkes einerseits und der Richtung des Armstützelementes, also der Richtung zwischen der Armschale, in die der Arm eingelegt wird, und dem Drehpunkt des Schwenkgelenkes zwischen Armstützelement und Kraftübertragungselement.

Vorzugsweise weist das Kraftübertragungselement wenigstens ein erstes Bauteil und ein zweites Bauteil auf, die durch ein Gelenk miteinander verbunden sind. Besonders vorteilhafterweise handelt es sich bei dem ersten Bauteil und/oder dem zweiten Bauteil um Stangen oder Stäbe, die durch ein Scharnier oder Schwenkgelenk miteinander verbunden sind, das eine einzige Schwenkachse aufweist. Durch das Kraftübertragungselement, das Teil des Gegenlagers ist, ist vorteilhafterweise eine Druckkraft übertragbar. Das Armstützelement soll durch eine Kraft, die von unten wirkt, unterstützt werden. Das Kraftübertragungselement soll die entsprechende Gegenkraft in das Gegenlagerelement übertragen, so dass es Druckkräfte übertragen können muss. Ist dies gewährleistet, wird durch die gelenkige Anordnung der beiden Bauteile des Kraftübertragungselementes aneinander erreicht, dass eine optimale Ausgestaltung des Kraftübertragungselementes je nach Bewegung und/oder Position des Benutzers erreicht wird. Die beiden Bauteile des Kraftübertragungselementes können sich relativ zueinander neu ausrichten, indem eines der Bauteile relativ zum anderen der Bauteile um das Schwenkgelenk herum verschwenkt wird. Dadurch können Abstände zwischen dem Gegenlagerelement und dem am Stützelement verändert und an die jeweilige Position und/oder Bewegung des Benutzers angepasst werden.

Besonders bevorzugt sind das erste Bauteil und/oder das zweite Bauteil längenveränderlich ausgebildet. Dabei kann das jeweils längenveränderliche Bauteil als Teleskopstange ausgebildet sein. Auf diese Weise lässt sich die Vorrichtung besonders leicht an unterschiedlich große Benutzer anpassen, indem durch die Teleskopierbarkeit oder die Längenveränderbarkeit wenigstens eines der Bauteile auch die Gesamtlänge des Kraftübertragungselementes angepasst werden kann. Besonders bevorzugt ist das längenveränderliche erste Bauteil und/oder das längenveränderliche zweite Bauteil in ihrer Länge veränderbar in unterschiedlichen Positionen, vorzugsweise stufenlos, jedoch auch arretierbar, so dass die einmal gewünschte Länge eingestellt werden kann und dann nicht mehr veränderlich ist, ohne einen entsprechenden Feststellmechanismus zu lösen.

Vorteilhafterweise handelt es sich bei dem Gelenk um ein Schwenkgelenk mit einer Schwenkachse.

In einer besonders bevorzugten Ausgestaltung sind das erste Bauteil und/oder das zweite Bauteil derart mit dem Gelenk verbunden, dass eine Bewegung des jeweiligen Bauteils um seine Längsachse relativ zu dem Gelenk möglich ist. Vorteilhafterweise befindet sich am oberen Ende eines der beiden Bauteile, das dem Gegenlagerelement abgewandt ist, das Armstützelement. Das Armstützelement und insbesondere ein Abstandselement des Armstützelementes ist vorteilhafterweise schwenkbar an dem jeweiligen Bauteil des Kraftübertragungselementes angeordnet. Wird dieses Bauteil, an dem sich das Armstützelement befindet, relativ zum Gelenk zwischen dem ersten Bauteil und dem zweiten Bauteil um seine Längsachse drehbar ausgestaltet, kann auch das Armstützelement drehbar um die Längsachse des jeweiligen Bauteils ausgebildet sein. Es werden damit weitere Bewegungen möglich, so dass die Position des Armstützelementes und insbesondere die Erstreckung und gegebenenfalls Form des jeweiligen Kraftübertragungselementes an die Position und/oder Bewegung des Benutzers der Vorrichtung angepasst werden kann.

Vorteilhafterweise ist das Kraftübertragungselement drehbar und/oder schwenkbar an dem Gegenlagerelement angeordnet. Dazu kann das Kraftübertragungselement beispielsweise an dem Gegenlagerelement durch ein Scharnier, ein Kugelgelenk oder ein sonstiges Gelenk befestigt oder beispielsweise mit einem Ende des Kraftübertragungselementes in eine dafür vorgesehene Tasche oder Halterung am Gegenlagerelement eingesteckt oder eingeführt sein. In einer besonders bevorzugten Ausgestaltung ist folglich eines der Bauteile des Kraftübertragungselementes drehbar und/oder schwenkbar am Gegenlagerelement angeordnet und das jeweilige Bauteil um seine eigene Längsachse drehbar relativ zu dem Gelenk angeordnet, durch das das jeweilige Bauteil mit dem jeweils anderen Bauteil des Kraftübertragungselementes verbunden ist. Besonders bevorzugt ist auch dieses andere Bauteil um seine eigene Längsachse drehbar am Gelenk zwischen den beiden Bauteilen befestigt. Dadurch wird eine umfassende Bewegung der Bauteile des Kraftübertragungselementes relativ zueinander, relativ zum Gegenlagerelement und vorzugsweise auch relativ zum Armstützelement möglich. Das Armstützelement verfügt über die Armschiene, in die der Arm eingelegt wird. Diese Armschiene lässt sich durch die vielseitig positionierbare und drehbare sowie verschwenkbare Ausgestaltung des Kraftübertragungselementes in eine Vielzahl unterschiedlicher Positionen bringen, wodurch nicht nur eine Bewegung des eigentlichen Schultergelenkes, also eines Kugelgelenkes, gefolgt werden kann, sondern auch andere Bewegungen möglich werden, die eine Bewegung der gesamten Schulter zur Folge haben.

Vorzugsweise ist das Gegenlagerelement ein Anlageelement zum Anlegen an den Rumpf des Benutzers. Es kann ein Gurt, ein Gürtel, eine Bandage oder ein Schalenelement sein und kann vorteilhafterweise in ein Kleidungsstück, beispielsweise eine Hose oder eine Weste integriert sein. Alternativ oder zusätzlich dazu weist das Gegenlagerelement ein Schulterelement zum Anlegen an einer Schulter des Benutzers auf.

Vorzugsweise weist das Kraftübertragungselement mehrere, vorzugsweise flächig ausgebildete, Teilelemente auf, die gegeneinander verschieblich ausgebildet sind. Diese Teilelemente sind vorzugsweise gebogen und weisen besonders bevorzugt zumindest abschnittsweise den gleichen Krümmungsradius auf. Sie können beispielsweise kreisbogenförmig ausgebildet sein. Sie weisen eine erste Erstreckungsrichtung auf, die größer als eine senkrecht dazu verlaufende zweite Erstreckungsrichtung ist. Entlang dieser ersten Erstreckungsrichtung sind die Teilelemente gegeneinander verschieblich ausgebildet, so dass sich durch Verschieben der wenigstens zwei Teilelemente die Länge und ein Gesamt-Krümmungswinkel des Kraftübertragungselementes einstellen lassen. Ist das Kraftübertragungselement aus mehr als zwei, beispielsweise drei, vier oder fünf Teilelementen aufgebaut, die jeweils paarweise aneinander verschieblich angeordnet sind, lässt sich ein Gesamt-Krümmungswinkel des Kraftübertragungselementes zumindest bereichsweise, vorzugsweise jedoch vollständig, unabhängig von der Länge des Kraftübertragungselementes einstellen. Dazu ist es von Vorteil, wenn die verschiedenen Teilelemente in den Bereichen, in denen sie aneinander anliegen und relativ zueinander verschiebbar sind, den gleichen Krümmungsradius, ansonsten jedoch unterschiedliche Kürmmungsradien aufweisen.

Die Teilelemente sind vorzugsweise aus einem Metall, beispielsweise Stahl, oder einem Kunststoff oder einem faserverstärkten Kunststoff, insbesondere kohlefaserverstärktem Kunststoff ausgebildet.

Mit Hilfe der beigefügten Zeichnungen werden nachfolgend einige Ausführungsbeispiele der vorliegenden Erfindung näher erläutert. Es zeigt:
- Figur 1: - die schematische Darstellung eines ersten Ausführungsbeispiels der vorliegenden Erfindung,
- Figur 2: - die schematische Darstellung eines weiteren Ausführungsbeispiels der vorliegenden Erfindung,
- Figur 3: - die schematische Darstellung eines nicht erfindungsgemäßen Ausführungsbeispiels,
- Figur 4: - die schematische Darstellung eines nicht erfindungsgemäßen Ausführungsbeispiels im angelegten Zustand
- Figur 5: - eine weitere schematische Darstellung eines nicht erfindungsgemäßen Ausführungsbeispiels,
- Figur 6: - die schematische Darstellung eines weiteren nicht erfindungsgemäßen Ausführungsbeispiels
- Figur 7: - die schematische Darstellung eines weiteren nicht erfindungsgemäßen Ausführungsbeispiels
- Figur 8: - eine vergrößerte Darstellung eines Teils der Figur 7 und
- Figuren 9 und 10: - die schematische Darstellung eines Kraftübertragungselementes aus drei Teilelementen.

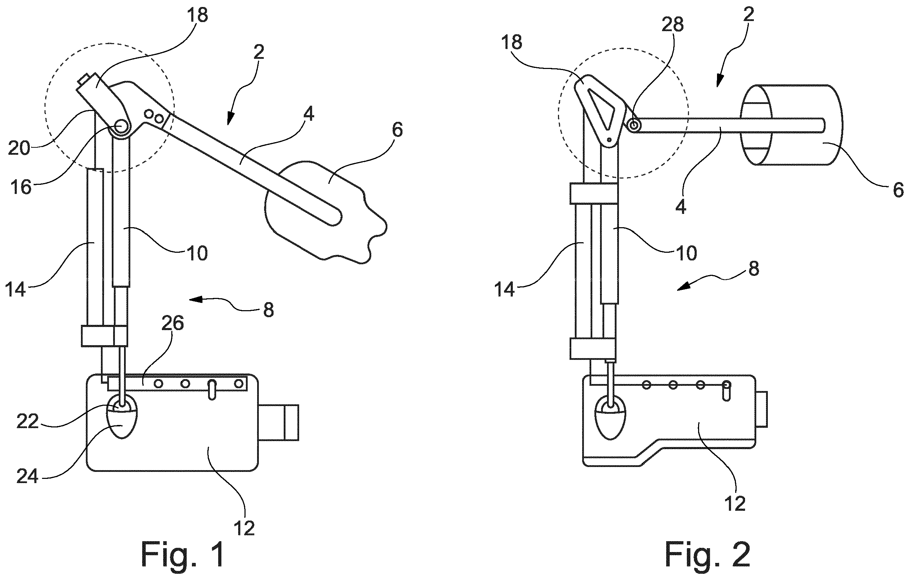

Figur 1 zeigt eine Vorrichtung zum Unterstützen eines Armes des Benutzers, die ein Armstützelement 2 mit einem Abstandselement 4 sowie eine Armschale 6 aufweist und über ein Gegenlager 8 verfügt, das im gezeigten Ausführungsbeispiel ein Kraftübertragungselement 10 sowie ein Gegenlagerelement 12 aufweist. Die Vorrichtung verfügt zudem über einen passiven Aktuator 14 in Form eines elastischen Elementes.

Das Armstützelement 2 ist um eine Schwenkachse 16, die auch Drehachse genannt werden kann, herum schwenkbar am Kraftübertragungselement 10 des Gegenlagers 8 befestigt. Drehfest am Armstützelement 2 ist zudem ein Kraftangriffshebel 18 angeordnet, an dem sich ein Kraftangriffspunkt 20 befindet, an dem die Kraft, die durch den passiven Aktuator 14 ausübbar ist, angreift.

Der Kraftangriffshebel 18 ist in zwei Zustände bringbar. Er ist im gezeigten Ausführungsbeispiel im ersten Zustand dargestellt, indem er drehfest am Armstützelement 2 und insbesondere am Abstandselement 4 angeordnet ist. Er ist jedoch in einen zweiten Zustand bringbar, indem er relativ zum Abstandselement 4 des Armstützelementes 2 verschwenkbar angeordnet ist. Auf diese Weise lässt sich ein Winkel zwischen dem Kraftangriffshebel 18 und dem Armstützelement 2 einstellen. Der Winkel wird dabei gebildet zwischen zwei Richtungen. Die eine Richtung ist die Richtung des Kraftangriffshebels 18, die die Richtung zwischen dem Kraftangriffspunkt 20 und der Schwenkachse 16 ist. Die zweite Richtung, die zum Bestimmen des Winkels benötigt wird, ist die Richtung des Armstützelementes 2. Dabei handelt es sich um die Richtung zwischen der Armschale 6, in der der Arm eingelegt wird, und der Schwenkachse 16.

Das Kraftübertragungselement 10 ist mit einem unteren Ende 22, das im gezeigten Ausführungsbeispiel einen Lagerpunkt bildet, in einer Tasche 24 positioniert, die am Gegenlagerelement 12 angeordnet ist. Das untere Ende des passiven Aktuators 14 ist über ein Zugelement 26 am Gegenlagerelement 12 angeordnet. Auf diese Weise wird eine vom passiven Aktuator 14 auf den Kraftangriffshebel 18 übertragene Kraft über das Gegenlager 8 auf den Körper des Benutzers übertragen. Das untere Ende 22 ist in der Tasche 24 schwenkbar und drehbar, so dass eine optimale Position des Gegenlagers 8 für jede Position des Arms gefunden werden kann.

Figur 2 zeigt eine alternative Ausgestaltung. Auch sie verfügt über ein Armstützelement 2 mit einem Abstandselement 4 und einer Armschale 6, sowie über ein Gegenlager 8 mit Kraftübertragungselement 10 und Gegenlagerelement 12 sowie über einen passiven Aktuator 14. Im Unterschied zu der in Figur 1 gezeigten Ausführungsform ist zwischen dem Abstandselement 4 des Armstützelementes 2 und dem Kraftangriffshebel 18 ein weiteres Schwenkgelenk 28 angeordnet, das schwenkbar aber feststellbar ausgebildet ist. Durch die Veränderung des Winkels des Schwenkgelenkes lässt sich ein Winkel zwischen dem Kraftangriffshebel 18 und dem Abstandselement 4 einstellen, so dass auch hier die Position des Armes und damit der Armschale 6 einstellbar ausgebildet ist, in der die durch den passiven Aktuator 14 auf den Kraftangriffshebel 18 übertragene Kraft und damit auch die Unterstützungskraft maximal ist.

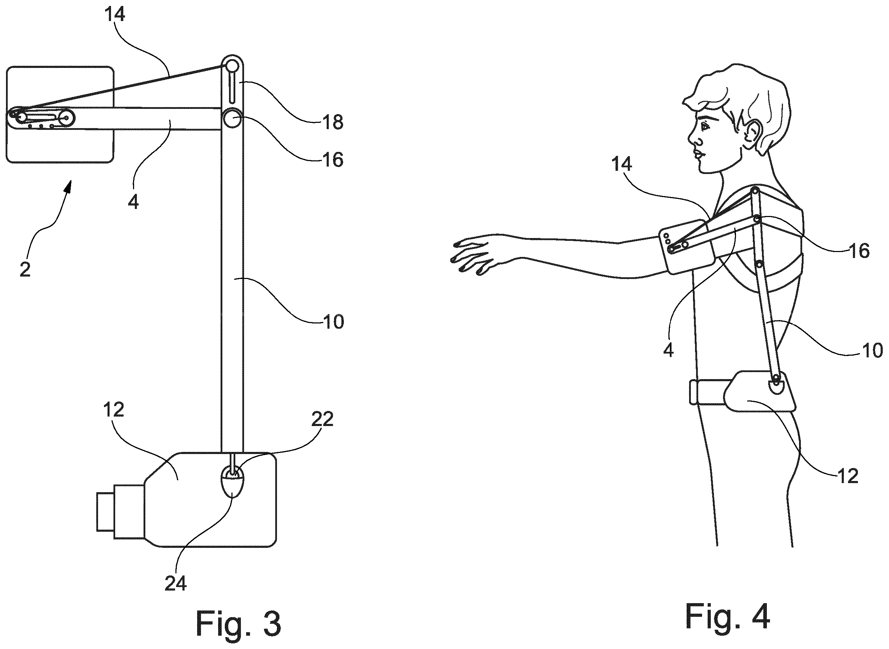

Figur 3 zeigt ein nicht erfindungsgemäßes Ausführungsbeispiel. Anders als bei den in den Figuren 1 und 2 gezeigten Ausführungsformen ist hier der Kraftangriffshebel 18 drehfest mit dem Kraftübertragungselement 10 verbunden. Der passive Aktuator 14 ist in Form eines elastischen Elementes zwischen dem Kraftangriffshebel 18 und einem Element des Armstützelementes 2 angeordnet. Das Abstandselement 4 des Armstützelementes 2 ist wieder um die Schwenkachse 16 herum schwenkbar am Kraftübertragungselement 10 angeordnet. Dessen unteres Ende 22 ist wieder in der Tasche 24 des Gegenlagerelementes 12 positioniert.

Ein Winkel zwischen dem Kraftangriffshebel 18 und dem Kraftübertragungselement 10 ist im gezeigten Ausführungsbeispiel einstellbar und feststellbar ausgebildet. Auf diese Weise lässt sich insbesondere die Position des Armes und damit des Armstützelementes 2 einstellen, in der die von dem passiven Aktuator 14 aufgebrachte Kraft maximal ist.

Figur 4 zeigt eine dem Ausführungsbeispiel aus Figur 3 ähnliche Ausgestaltung im angelegten Zustand. Das Gegenlagerelement 12 ist um die Hüfte des Trägers herum angeordnet. Das Abstandselement 4 des Armstützelementes 2 ist um die Schwenkachse 16 herum am Kraftübertragungselement 10 schwenkbar angeordnet. Der passive Aktuator 14 ist wie in Figur 3 ausgebildet.

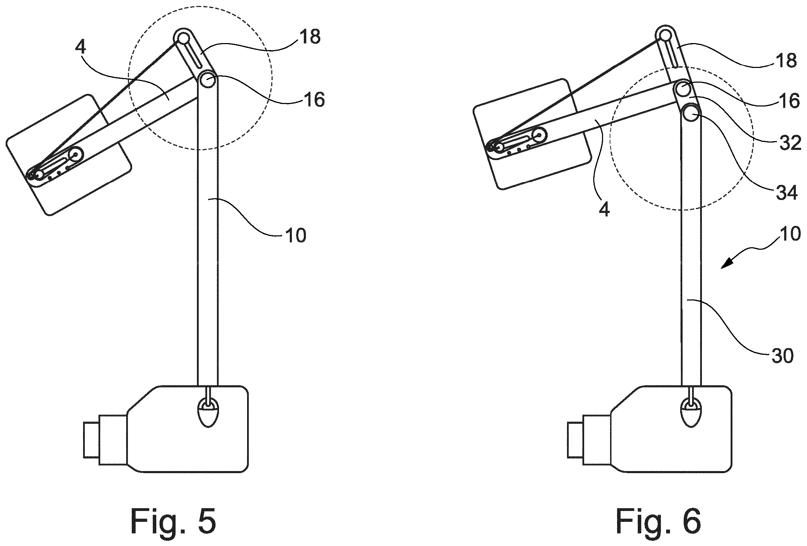

Figur 5 zeigt die Ausgestaltung aus Figur 3 im verschwenkten Zustand. Man erkennt, dass ein Winkel zwischen dem Abstandselement 4 des Armstützelementes 2 sowie dem Kraftübertragungselement 10 um die Schwenkachse 16 herum verändert wurde. Durch die erfindungsgemäße Einstellbarkeit des Winkels ist es möglich, den Kraftangriffshebel 18, der im gezeigten Ausführungsbeispiel drehfest mit dem Kraftübertragungselement 10 verbunden ist, in die gezeigte Position zu bringen, in dem auch er relativ zum Kraftübertragungselement 10 um die Schwenkachse 16 herum verschwenkt wird. Dazu ist es von Vorteil, wenn der Kraftangriffshebel 18 in einen ersten Zustand und in einen zweiten Zustand bringbar ist, wobei er im ersten Zustand relativ zum Kraftübertragungselement 10 und relativ zum Abstandselement 4 verschwenkbar ist.

Figur 6 zeigt ein weiteres nicht erfindungsgemäßes Ausführungsbeispiel. Das Kraftübertragungselement 10 verfügt über ein erstes Bauteil 30 sowie über ein zweites Bauteil 32, die um ein Schwenkgelenk 34 schwenkbar aneinander angeordnet sind. Dabei ist ein Winkel zwischen dem ersten Bauteil 30 und dem zweiten Bauteil 32 über das Schwenkgelenkt 34 einstellbar und feststellbar, so dass eine einmal gefundene Position über das Schwenkgelenk 34 arretiert werden kann. Das Abstandselement 4 des Armstütz-elementes 2 ist um die Schwenkachse 16 herum relativ zum zweiten Bauteil 32 verschiebbar angeordnet.

Da der Kraftangriffshebel 18 relativ zum zweiten Bauteil 32 drehfest angeordnet ist, lässt sich durch Verschwenken des zweiten Bauteils 32 relativ zum ersten Bauteil 30 um das Schwenkgelenk 34 herum auch der Winkel zwischen dem Kraftangriffshebel 18 und dem ersten Bauteil 30 einstellen.

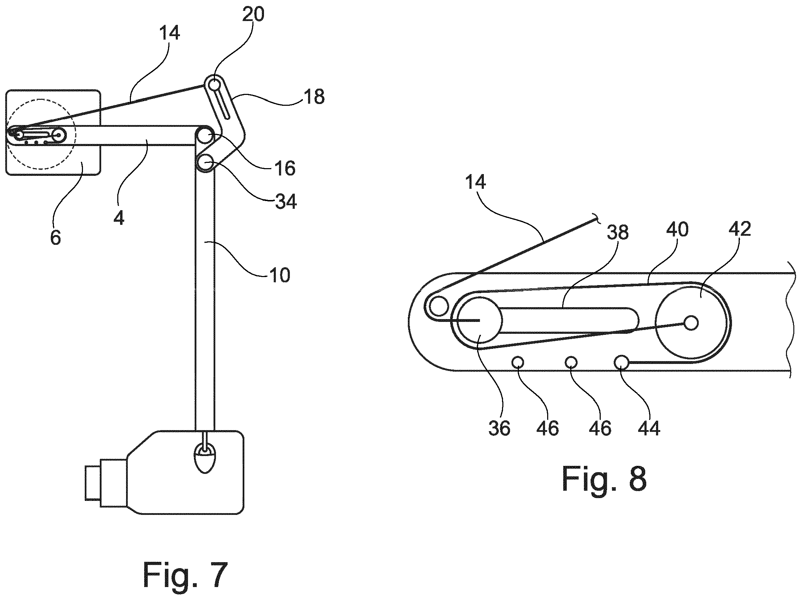

Ein weiteres nicht erfindungsgemäßes Ausführungsbeispiel ist in Figur 7 dargestellt. Auch hier ist wieder das Abstandselement 4 des Armstützelementes 2 relativ zum Kraftübertragungselement 10 um die Schwenkachse 16 herum schwenkbar. Der Kraftangriffshebel 18 ist drehfest aber einstellbar mit dem Kraftübertragungselement 10 verbunden. Dafür ist ein Schwenkgelenk 34 vorhanden, so dass der Winkel einstellbar ist. Der passive Aktuator 14 erstreckt sich von einem Kraftangriffspunkt 20 bis zu einem Element im Bereich der Armschale 6. Figur 8 zeigt einen vergrößerten Bereich. Man erkennt den passiven Aktuator 14, der an einer gleitbaren Rolle 36 angeordnet ist. Diese kann in einem Langloch 38 verschoben werden, indem ein Seilelement 40 verändert wird. Ein erstes Ende des Seilelementes 40 ist an einem Scheibenelement 42 angeordnet, das Teil des Armstützelementes 2 ist. Das Seilelement 40 verläuft um die Rolle 36 und das Scheibenelement 42 herum und wird im gezeigten Ausführungsbeispiel durch einen Pin 44, der in ein Loch 46 gesteckt ist, befestigt.

Wird der Pin 44 aus dem in Figur 8 gezeigten Zustand gelöst und in eines der weiter links angeordneten Löcher 46 eingesteckt, führt dies dazu, dass die Rolle 36 im Langloch 38 im gezeigten Ausführungsbeispiel nach rechts verschoben wird. Dadurch wird der passive Aktuator 14 gespannt und so die Kraft, die auf den Kraftangriffshebel 18 wirkt, erhöht.

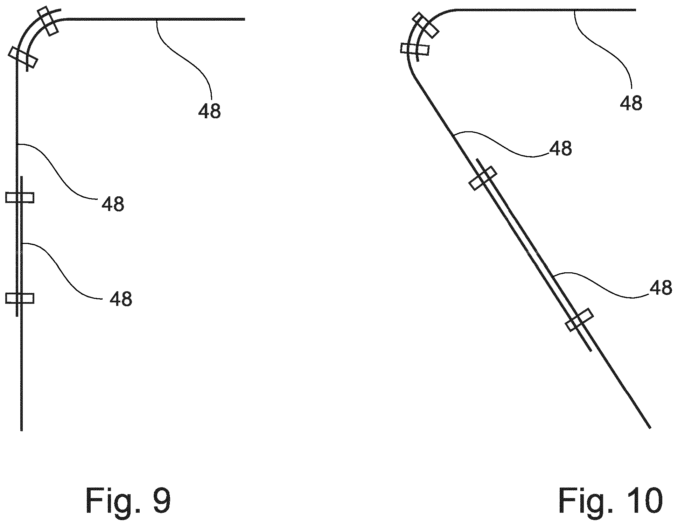

Die Figuren 9 und 10 zeigen jeweils ein Kraftübertragungselement 10, das aus jeweils drei Teilelementen 48 aufgebaut ist. Die drei Verteilelemente 48 sind über jeweils zwei Verbindungselemente paarweise miteinander gekoppelt und verfügen in diesem Bereich über den gleichen Krümmungsradius. Sie können somit gegeneinander verschoben werden. Dadurch lassen sich die in den Figuren 9 und 10 dargestellten unterschiedlichen Krümmungsradien zwischen den beiden Richtungen erreichen, in die sich das Kraftübertragungselement an seinen Enden erstreckt. Insbesondere durch die Verschiebung der unteren beiden Teilelemente 48, die im aneinander anliegenden Bereich gerade ausgebildet sind, lässt sich die Länge des Kraftübertragungselementes einstellen.

### Bezugszeichenliste

- 2: Armstützelement
- 4: Abstandselement
- 6: Armschale
- 8: Gegenlager
- 10: Kraftübertragungselement
- 12: Gegenlagerelement
- 14: passiver Aktuator
- 16: Schwenkachse
- 18: Kraftangriffshebel
- 20: Kraftangriffspunkt
- 22: unteres Ende
- 24: Tasche
- 26: Zugelement
- 28: Schwenkgelenk
- 30: erstes Bauteil
- 32: zweites Bauteil
- 34: Schwenkgelenk
- 36: Rolle
- 38: Langloch
- 40: Seilelement
- 42: Scheibenelement
- 44: Pin
- 46: Loch
- 48: Teilelement

## Patentansprüche

1. Vorrichtung zum Unterstützen wenigstens eines Armes eines Benutzers, wobei die Vorrichtung
a. wenigstens ein Armstützelement (2, 4) mit je einer Armschale (6) zum Anlegen an jeweils einen Arm,
b. wenigstens einen passiven Aktuator (14),
i. der eingerichtet ist, eine Kraft auf wenigstens eines der Armstützelemente (2, 4) auszuüben, und
c. wenigstens ein Gegenlager (8) für die aufzubringende Kraft aufweist,
i. das wenigstens ein Kraftübertragungselement (10) und ein Gegenlagerelement (8) aufweist,
wobei die Vorrichtung wenigstens einen Kraftangriffshebel (18) aufweist, der mit einem der Armstützelemente (2, 4) drehfest verbunden ist und mit diesem einen Winkel einschließt und an dem der wenigstens eine passive Aktuator (14) angreift,
**dadurch gekennzeichnet, dass**
der Kraftangriffshebel (18) in einen ersten Zustand, in dem er relativ zu dem Armstützelement (2, 4) und dem Kraftübertragungselement (10) schwenkbar ist, so dass der Winkel einstellbar ist, indem der Kraftangriffshebel verschwenkt wird, und in einen zweiten Zustand bringbar ist, in dem er drehfest an dem Armstützelement (2, 4) angeordnet ist, so dass die Position des Arms des Benutzers veränderbar ist, in der der Kraftangriffshebel (18) senkrecht auf der durch den wenigstens einen Aktuator (14) aufbringbaren Kraft steht,
wobei sich beim Anheben des Arms des Benutzers der Winkel zwischen dem Kraftangriffshebel (18) und dem Kraftübertragungselement (10) verändert und sich gleichzeitig der Winkel zwischen dem Kraftangriffshebel (18) und der Wirkrichtung der durch den wenigstens einen Aktuator (14) aufbringbaren Kraft verändert.

2. Vorrichtung nach Anspruch 1, **dadurch gekennzeichnet, dass** die Vorrichtung zwei Armstützelemente (2, 4) mit je einer Armschale (6) zum Anlegen an jeweils einen Arm des Benutzers aufweist.

3. Vorrichtung nach einem der vorstehenden Ansprüche, **dadurch gekennzeichnet, dass** das Kraftübertragungselement (10) und/oder das Armstützelement (2, 4) zwei Bauteile (30, 32) aufweist, die durch ein Schwenkgelenk (28) verbunden sind, so dass ein Winkel zwischen diesen Bauteilen (30, 32) einstellbar ist.

4. Vorrichtung nach Anspruch 3, **dadurch gekennzeichnet, dass** das Schwenkgelenk (26) lösbar und feststellbar ist.

5. Vorrichtung nach einem der vorstehenden Ansprüche, **dadurch gekennzeichnet, dass** ein Kraftangriffspunkt (20), an dem der Aktuator (14) an dem Kraftangriffshebel (18) angreift, verschiebbar ist.

6. Vorrichtung nach einem der Ansprüche 3 bis 5, **dadurch gekennzeichnet, dass** das erste Bauteil (30) und/oder das zweite Bauteil (32) längenveränderlich ausgebildet sind, insbesondere Teleskopstangen sind.

7. Vorrichtung nach einem der Ansprüche 3 bis 6, **dadurch gekennzeichnet, dass** das erste Bauteil (30) und/oder das zweite Bauteil (32) derart mit dem Gelenk verbunden sind, dass eine Bewegung des jeweiligen Bauteils (30, 32) um seine Längsachse relativ zu dem Gelenk möglich ist.

8. Vorrichtung nach einem der vorstehenden Ansprüche, **dadurch gekennzeichnet, dass** das Kraftübertragungselement (10) drehbar und/oder schwenkbar an dem Gegenlagerelement (12) angeordnet ist.

## Claims

1. A device for supporting at least one arm of a user, wherein the device has
a. at least one arm support element (2,4), each of which has an arm shell (6) for placing on an arm,
b. at least one passive actuator (14),
i. which is configured to apply a force to at least one of the arm support elements (2,4), and
c. at least one counter bearing (8) for the force to be applied,
i. which comprises at least one force transmission element (10) and a counter bearing element (8),
wherein the device features at least one force application lever (18), which is connected to one of the arm support elements (2,4) such that it is torque-proof and confines an angle, and with which the at least one passive actuator (14) engages,
**characterized by the fact that**
the force application lever (18) can be brought into a first state, in which it can be swivelled relative to the arm support element (2,4) and the force transmission element (10), and a second state, in which it is arranged with the arm support element (2,4) such that it is torque-proof, such that the position of the arm of the user is adjustable, in which the force application lever (18) is perpendicular to acting force exerted by the actuator (14), wherein when the user raises their arm, angle between the force application lever (18) and the force transmission element (14) changes and at the same time, the angle between the force application lever (18) and the effective direction of the force that can be exerted by the at least one passive actuator (14) change.

2. The device according to claim 1, **characterized by** the fact that the device comprises two arm support elements (2,4), each of which has an arm shell (6) for placing on one arm of the user.

3. The device according to one of the above claims, **characterized by** the fact that the force transmission element (10) and/or the arm support element (2,4) features two structural components (30,32) that are connected by a swivel joint (28), such that an angle between these structural components (30,32) is adjustable.

4. The device according to claim 3, **characterized by** the fact that the swivel joint (26) can be released and tightened.

5. The device according to one of the above claims, **characterized by** the fact that a force application point (20), at which the actuator (14) engages with the force application level (18), can be displaced.

6. The device according to one of the claims 3 to 5, **characterized by** the fact that the first structural component (30) and/or the second structural component (32) are designed to be adjustable in length; specifically, they are designed to be telescopic rods.

7. The device according to one of the claims 3 to 6, **characterized by** the fact that the first structural component (30) and/or the second structural component (32) are connected to the joint such that a movement of the respective structural component (30,32) about its longitudinal axis relative to the joint is possible.

8. The device according to one of the above claims, **characterized by** the fact that a force application element (10) is arranged on the counter bearing element such that it can be rotated and/or swivelled.

## Revendications

1. Dispositif destiné à soutenir au moins un bras d'un utilisateur, le dispositif comprenant
a. au moins un élément de soutien de bras (2, 4) ayant chacun une coque pour bras (6) destinée à être appliquée contre un bras respectif,
b. au moins un actionneur passif (14),
i. qui est conçu pour exercer une force sur l'un au moins des éléments de soutien de bras (2, 4), et
c. au moins un support antagoniste (8) pour la force à appliquer,
i. qui comprend au moins un élément de transmission de force (10) et un élément de support antagoniste (8),
le dispositif comprenant au moins un levier d'application de force (18) qui est relié solidairement en rotation à l'un des éléments de soutien de bras (2, 4) et qui définit un angle avec ce dernier et sur lequel agit ledit au moins un actionneur passif (14),
**caractérisé en ce que**
le levier d'application de force (18) peut être amené dans un premier état, dans lequel il peut pivoter par rapport à l'élément de soutien de bras (2, 4) et à l'élément de transmission de force (10), de sorte que l'angle est réglable par pivotement du levier d'application de force, et dans un deuxième état, dans lequel il est disposé solidairement en rotation sur l'élément de soutien de bras (2, 4), de sorte que la position du bras de l'utilisateur peut être modifiée, dans laquelle le levier d'application de force (18) est perpendiculaire à la force susceptible d'être exercée par ledit au moins un actionneur (14),
lorsque l'utilisateur lève son bras, l'angle entre le levier d'application de force (18) et l'élément de transmission de force (10) varie, et, en même temps, l'angle entre le levier d'application de force (18) et la direction d'action de la force, susceptible d'être exercée par ledit au moins un actionneur (14), varie.

2. Dispositif selon la revendication 1,
**caractérisé en ce que** le dispositif comprend deux éléments de soutien de bras (2, 4) ayant chacun une coque pour bras (6) destinée à être appliquée contre un bras respectif de l'utilisateur.

3. Dispositif selon l'une des revendications précédentes,
**caractérisé en ce que** l'élément de transmission de force (10) et/ou l'élément de soutien de bras (2, 4) présente deux composants (30, 32) qui sont reliés par une articulation pivotante (28), de sorte qu'un angle entre ces composants (30, 32) est réglable.

4. Dispositif selon la revendication 3,
**caractérisé en ce que** l'articulation pivotante (26) peut être déverrouillée et verrouillée.

5. Dispositif selon l'une des revendications précédentes,
**caractérisé en ce qu'**un point d'application de force (20), au niveau duquel l'actionneur (14) agit sur le levier d'application de force (18), est déplaçable.

6. Dispositif selon l'une des revendications 3 à 5,
**caractérisé en ce que** le premier composant (30) et/ou le deuxième composant (32) sont réalisés de façon variable en longueur, en particulier sous forme de tiges télescopiques.

7. Dispositif selon l'une des revendications 3 à 6,
**caractérisé en ce que** le premier composant (30) et/ou le deuxième composant (32) sont reliés à l'articulation de manière à permettre un mouvement du composant respectif (30, 32) autour de son axe longitudinal par rapport à l'articulation.

8. Dispositif selon l'une des revendications précédentes,
**caractérisé en ce que** l'élément de transmission de force (10) est disposé de façon mobile en rotation et/ou en pivotement sur l'élément de support antagoniste (12).
